(19) Europäisches Patentamt
European Patent Office
Office européen des brevets

(11) EP 1 027 889 B1

(12) EUROPÄISCHE PATENTSCHRIFT

(45) Veröffentlichungstag und Bekanntmachung des Hinweises auf die Patenterteilung:
**07.12.2005 Patentblatt 2005/49**

(51) Int Cl.[7]: **A61K 9/70**, A61K 31/48

(21) Anmeldenummer: **00250117.9**

(22) Anmeldetag: **14.05.1992**

# (54) Mittel zur transdermalen Applikation enthaltend Ergolin-Derivate

Ergolin derivates for transdermal application

Dérivés de l' Ergolin à application transdermique

(84) Benannte Vertragsstaaten:
**AT BE CH DE DK ES FR GB GR IT LI LU NL SE**

(30) Priorität: **18.05.1991 DE 4116912**

(43) Veröffentlichungstag der Anmeldung:
**16.08.2000 Patentblatt 2000/33**

(62) Dokumentnummer(n) der früheren Anmeldung(en) nach Art. 76 EPÜ:
**92909703.8 / 0 538 433**

(73) Patentinhaber: **NeuroBiotec GmbH**
**10115 Berlin (DE)**

(72) Erfinder:
- **Riedl, Jutta, Dr.**
  **79639 Grenzach-Wyhlen (DE)**
- **Günther, Clemens, Dr.**
  **13357 Berlin (DE)**
- **Lipp, Ralph, Dr.**
  **14169 (DE)**

(74) Vertreter: **Wablat, Wolfgang et al**
**Wablat . Lange . Karthaus**
**Anwaltssozietät**
**Potsdamer Chaussee 48**
**14129 Berlin (DE)**

(56) Entgegenhaltungen:
**EP-A- 0 137 278** **EP-A- 0 155 229**
**WO-A-91/00746**

- **DATABASE EMBASE [Online] ELSEVIER SCIENCE PUBLISHERS, AMSTERDAM, NL; AGNOLI A. ET AL: "Dopamine receptor agonists as possible first-choice therapy of early Parkinson's disease." retrieved from STN Database accession no. 91321379 XP002153776 & NEW TRENDS IN CLINICAL NEUROPHARMACOLOGY, (1991) 5/2 (41-48). ,**
- **DATABASE EMBASE [Online] ELSEVIER SCIENCE PUBLISHERS, AMSTERDAM, NL; TSUI J.K.C.: "Future treatment of Parkinson's disease." retrieved from STN Database accession no. 92118765 XP002153777 & CANADIAN JOURNAL OF NEUROLOGICAL SCIENCES, (1992) 19/1 SUPPL. (160-162). ,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 027 889 B1

## Beschreibung

Die Erfindung betrifft ein Mittel zur transdermalen Applikation, welches dadurch gekennzeichnet ist, daß es Lisurid in Kombination mit einem oder mehreren penetrationsverstarkenden Mittel(n) enthält.

Ergolin-Derivate, wie das bereits seit 1960 bekannte Lisurid [3-(9,10-Dihydro-6-methyl-8α-ergolinyl-1,1-diethylharnstoff] und dessen Derivate, die unter anderen in der DE-A 2 238 540, der EP-A 002:206, der EP-A 0056358 und der EP-A 0160840 beschrieben sind, sind pharmakologisch wirksame Substanzen, die zur Herstellung von Arzneimitteln verwendet werden können.

Derartige Ergolin-Derivate sind beispielsweise solche der allgemeinen Formel

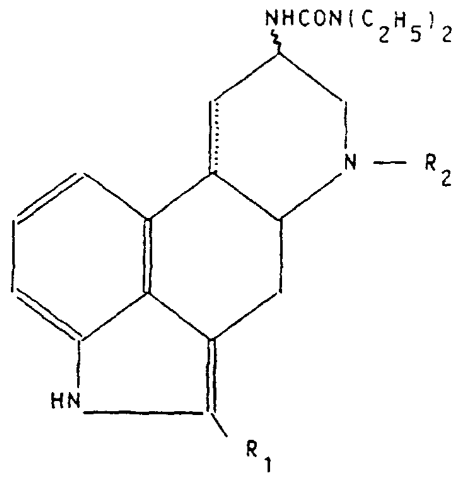

worin

⋯⋯eine Einfachbindung oder eine Doppelbindung symbolisiert,

$R_1$ ein Wasserstoffatom, ein Halogenatom oder eine Alkylthiogruppe mit 1 bis 4 Kohlenstoffatomen im Alkylrest darstellt und

$R_2$ eine Alkylgruppe mit 1 bis 4 Kohlenstoffatomen bedeutet, oder deren Salz mit einer physiologisch unbedenklichen Saure.

Als hochwirksame Ergolin-Derivate seien neben dem Lisurid das Bromlisurid [=3-(2-Brom-9,10-dehydro-6-methyl-8α-ergolinyl-1,1-diethylharnstoff], das Tergurid [=3-(6-Methyl-8α-ergolinyl-1,1-diethylharnstoff] und das Protergurid [=3-(6-Propyl-8α-ergolinyl)-1,1-diethylharnstoff] erwähnt.

Als Salze dieser Ergolin-Derivate seien die Sulfate, Phosphate, Maleate, Citrate oder Succinate erwähnt.

Ergolin-Derivate haben heute als Dopaminagonisten einen festen Platz in der Therapie verschiedenster Erkrankungen, die durch Hyperprolaktinamie bedingt sind oder bei denen Prolaktin von pathogenetischer Bedeutung ist.

Für primäres und sekundäres Abstillen sind Dopaminagonisten die Mittel der Wanl, ebenso fur die mit Hyperprolaktinamie eingehenden Fertilitatsstörungen der Frau. Auch durch Hyperprolaktinämie bedingte Potenzstörungen des Mannes können erfolgreich behandelt werden. Während Dopamin und Dopaminagonisten beim Gesunden die Sekretion von STH stimulieren, haben sie bei der Akromegalie den gegenteiligen Effekt. Praemenstruelles Syndrom, Praeklampsie, Geriatrie, Mastodynie werden ebenfalls mit dem weiten Wirkungsspektrum von Prolaktin in Verbindung gebracht und können mit Dopaminagonisten behandelt werden. Dem Parkinsonismus liegt ein Dopaminmangel zugrunde, deshalb können Parkinsonpatienten erfolgreich mit Dopaminagonisten behandelt werden.

Viele Ergolin-Derivate, wie zum Beispiel das Lisurid selbst haben nur eine kurze terminale Halbwertzeit und es ist demzufolge schwierig über einen längeren Zeitraum hin im Blut konstante Plasmaspiegel des Medikaments zu erreichen.

Es wurde nun gefunden, daß Lisurid in Kombination mit einem oder mehreren penetrationsverstärkenden Mittel(n) sehr gut zur Herstellung eines Mittels für die transdermale Applikation des Wirkstoffes verwendet werden kann.

Transdermal zu applizierende Arzneimittel haben bekanntlich den Vorzug, daß sie über einen längeren Zeitraum hin eine gleichmäßigere Freisetzung des Wirkstoffs ermöglichen, als dies in der Regel bei anders - wie zum Beispiel oral - zu applizierenden Mitteln möglich ist. Diese Eigenschaften lassen sich in einer Reihe von Erkrankungen vorteilhaft ausnutzen. Für in üblichen Pflastermassen schwer lösliche Wirkstoffe, wie zum Beispiel die Ergolin-Derivate ist es aber in der Regel recht problematisch, transdermale Systeme zu erstellen, die eine zur Therapie ausreichende Penetration des Wirkstoffs durch die Haut gewährleisten.

Es wurde nun gefunden, daß es mit Hilfe des erfindungsgemäßen Mittels überraschenderweise möglich ist, eine therapeutisch ausreichende und sehr gleichmäßige Penetrationsgeschwindigkeit der Lisurid durch die Haut zu erzielen.

Zur Herstellung pnarmazeutischer Präparate kann der Wirkstoff in geeigneten flüchtigen Lösungsmitteln und/oder penetrationsverstärkenden Mitteln gelöst oder suspendiert werden. Die erhaltenen Lösungen oder Suspensionen können mit den üblichen Hilfsstoffen, wie zum Beispiel Verdickungsmitteln versetzt werden. Zu erwähnen ist ferner, daß die erfindungsgemäßen Lösungen oder Suspensionen beispielsweise mittels Siliconelastomeren zu wirkstoffhaltigen Pflastern oder Bandagen verarbeitet werden können (DE-A 31 31 610; US-A 3,996,934 oder US-A 4,336,243).

Geeignete flüchtige Lösungsmittel sind beispielsweise niedere Alkohole, Ketone oder niedere Carbonsäureester wie Ethanol, Isopropanol, Aceton oder Ethylacetat, polare Ether, wie Tetrahydrofuran, niedere Kohlenwasserstoffe, wie Cyclohexan oder Benzin oder auch Halogenkohlenwasserstoffe, wie Dichlormethan, Trichlormethan, Trichlortrifluorethan und Trichlorfluormethan. Es bedarf keiner Erläuterung, daß auch Gemische dieser Lösungsmittel geeignet sind.

Geeignete penetratzonsverstärkende Mittel sind beispielsweise flüssige, ein- oder mehrwertige allphatische, cycloaliphatische oder aromatischaliphatische Alkohole mit bis zu 8 Kohlenstoffatomen wie 1,2-Propandiol Menthol, Dexpanthenol oder Benzylalkohol, gesättigte und ungesättigte Fettalkohole mit 8 bis 18 Kohlenstoffatomen, wie Laurylalkohol, Isocetylalkohol oder Cetylalkohol, Kohlenwasserstoffe, wie Mineralöl, gesättigte und ungesättigte Fettsäuren mit 8 bis 18 Kohlenstoffatomen, wie Laurinsäure, Isopalmitinsäure, Isostearinsäure oder Olsäure.

Fettsäureester, die sich für das erfindungsgemäße Mittel eignen, sind beispielsweise solche der Laurinsäure, Myristinsäure, Stearinsäure und Palmitinsäure,wie zum Beispiel die Methylester, Ethylester, 2-Hydroxyethylester, Glycerolester, Propylester, Isopropylester, Butylester, sec.-Butylester, Isobutylester dieser Säuren. Besonders bevorzugte Ester sind solche der Palmitinsäure, Isopalmitinsäure, Isostearinsäure und Stearinsäure, insbesondere zur Herstellung Lisuridhaltiger Mittel zur transdermalen Applikation, wie deren Methylester und insbesondere deren Isopropylester. Geeeignete Dicarbonsäurediester sind beispielsweise das Diisopropyladipat, Diisobutyladipat und Diisopropylsebacat. Es bedarf keiner näheren Erläuterung, das auch Gemische dieser penetrationsverstarkenden Mittel zur Herstellung des erfindungsgemäßen Mittels geeignet sind.

Eine sehr gleichmäßige Applikation mit eingestellter Dosierung des Werkstoffes kann man erzielen wenn man den Wirkstoff in ein transdermales therapeutisches System (TTS) einbettet. Geeignete transdermale therapeutische Systeme sind solche, die man üblicherweise zur percutanen Applikation von Wirkstoffen anwendet (Yie W. Chien: "Tansdermal Controlled Systemic Medications", Marcel Dekker, Inc., New York and Basel, 1987, Dr. Richard Baker: "Analysis of Transdermal Drug Delivery Patents 1934 to 1984" und "Analysis of Recent Transdermal Delivery Patents, 1984-1986 and Enhancers" Membrane Technology & Reserch 1030 Hamilton Court Menlo Park CA 94025 (415) 328-2228).

So kann man beispielsweise ein solches transdermales therapeutisches System verwenden, welches aus

a) einer undurchlässigen Deckschicht,
einer an der Deckschicht haftenden, das Ergolin-Derivat und das oder die penetrationsverstärkende Mittel enthaltenden, für diese Komponenten durchlässigen Arzneimittelschicht, die selbsthaftend ist oder von einem Hauthaftkleber abgedeckt oder umgeben ist, welcher ebenfalls penetrationsverstärkende Mittel enthalten kann und
einer abziehbaren Schutzschicht, oder

b) einer undurchlässigen Deckschicht,
einem an oder in der Deckschicht befindlichen, das Ergolin-Derivat penetrationsverstärkende Mittel enthaltenden Arzneimittelreservoir,
für diese Komponenten durchlässigen Polymerschicht,
einer durchlässigen gegebenenfalls penetrationsverstärkende Mittel enthaltende Hauthaftkleberschicht und
einer abziehbaren Schutzschicht besteht.

Ein transdermales therapeutisches System gemäß Variante a stellt ein einfaches Matrixsystem dar. Es kann beispielsweise wie folgt hergestellt werden.

Eine Lösung oder Suspension von 1 bis 25 Gew. % Wirkstoff, 0-40 Gew. % eines penetrationsverstärkenden Mittels, 30-70 Gew. % eines medizinisch üblichen Klebers aufgefüllt mit einem geeigneten flüchtigen Lösungsmittels zu 100 Gew. % wird auf eine plane undurchlässige Deckschicht gestrichen und nach dem Trocknen mit einer abziehbaren Schutzschicht versehen.

Verwendet man einen medizinisch üblichen Matrixbildner, der nach dem Trocknen des Systems nicht oder nicht ausreichend auf der Haut haftet, so kann man das System vor dem Aufbringen der abziehbaren Schutzschicht noch zusätzlich mit einem Hauthaftkleber abdecken oder umgeben.

Geeignete Lösungsmittel und penetrationsverstärkenden Mittel sind beispielsweise die bereits erwähnten

Flüssigkeiten dieser Art. Als medizinisch übliche Kleber eignen sich beispielsweise Polyacrylate, Silicone, Polyurethane, sowie natürliche oder synthetische Kautschuke. Als weitere Matrixbildner kommen Celluloseether, Polyvinylverbindungen oder Silikate in Betracht.

Als Schutzschichten eignen sich alle Folien, die man üblicherweise bei transdermalen therapeutischen Systemen anwendet. Solche Folien sind beispielsweise silikonisiert oder fluorpolymerbeschichtet.

Als Deckschicht kann man bei diesem System beispielsweise 10 bis 100 µm dicke Folien aus Polyethylen oder Polyester wahlweise pigmentiert oder metallisiert verwenden. Die hierauf aufgebrachte Arzneimittelschicht hat vorzugsweise eine Dicke von 20 bis 500 µm. Die Abgabe der Wirkstoffe erfolgt vorzugsweise über eine Fläche von 5 bis 100 $cm^2$.

Ein transdermales therapeutisches System gemäß Variante b kann beispielsweise wie folgt hergestellt werden.

Eine undurchlässige Folie wird durch Wärme und/oder Zug so verformt, daß eine 0,1 bis 3 ml fassende Ausbuchtung entsteht. Diese wird mit einer wirkstoffhaltigen Lösung oder Suspension enthaltend 1-50 Gew. Z Wirkstoff in einem penetrationsverstärkenden Mittel gefüllt. Die wirkstoffhaltige Lösung oder Suspension kann auch mit bis zu 10 Gew. % Matrixbildner verdickt sein.

Als Abdeckung des Reservoirs zur Haut hin dient eine aufgeschweißte oder aufgeklebte durchlässige Polymerschicht, auf welche eine durchlässige Hauthaftkleberschicht und eine abziehbare Schutzschicht angebracht wird.

Es können bei diesem System die oben erwähnten penetrationsverstärkenden Mittel angewendet werden. Als durchlässige Polymerschicht wird beispielsweise eine 20 bis 200 µm dicke Folie aus Celluloseestern, Celluloseethern, Siliconen oder Polyolefinverbindungen verwendet. Durch Variation dieser Polymerschicht läßt sich die Diffusionsgeschwindigkeit des Wirkstoffes oder Wirkstoffgemisches innerhalb weiter Grenzen variieren.

Als Kleber und Schutzschicht eignen sich die gleichen Materialien, die bei dem transdermalen therapeutischen System gemäß Variante a beschrieben sind.

So lassen sich durch einfache Variation der verschiedenen Parameter transdermale therapeutische Systeme mit unterschiedlichen Freisetzungsraten des Wirkstoffes oder Wirkstoffgemisches herstellen, die zwecks Lagerung beispielsweise in Aluminiumfolien verpackt werden können.

Die Konzentration, in welcher das Ergolin-Derivat optimalerweise in der Penetrationsverstärkung gelöst oder suspendiert wird, ist naturgemäß von der Art des verwendeten Wirkstoffs und Penetrationsverstärker und der angestrebten Einzeldosis abhängig. Sie muß im Einzelfalle mittels der dem Fachmann geläufigen Vorversuche, wie zum Beispiel der Bestimmung der erreichbaren Blutplasmakonzentrationen an Wirkstoff bei ausgewählten erfindungsgemäßen Mitteln pro Fläche ermittelt werden. Im allgemeinen werden Wirkstoffkonzentrationen von 0.2 bis 20 Gewichtsprozent im erfindungsgemäßen Mittel ausreichend sein.

Die Bestimmung des Ausmaßes der Geschwindigkeit der percutanen Resorption durch die erfindungsgemäßen Mittel kann beispielsweise mittels radioaktiv markierter Ergolin-Derivate erfolgen.

Frisch bereitete, von subcutanem Fett befreite Haut vom Abdomen haarloser Mäuse wird in eine Franz Diffusionszelle eingespannt, die als Auffangflüssigkeit isotonische Polyethylenglykol-(MG 400)-Lösung oder Phosphat-Puffer-Lösung vom pH 7 enthält. Dann gibt man 2 µl Testlösung auf die Haut und ermittelt nach 24, 48 und 72 Stunden mittels Flüssigkeits-Scintilationszählung den Gehalt des in die Auffangflüssigkeit gelangten Ergolin-Derivats.

Getestet wurde eine 5 gewichtsprozentige Lösung des Ergolins in Propylenglycol (PG) und Propylenglycol-Laurinsäure 9:1 w/w (PG + 10 % LS).

Die nachfolgende Tabelle zeigt die in diesem Versuch erhaltenen Ergebnisse:

Tabelle

| Parameter | Dimension | Lisurid in PG + 10 % LS | |
|---|---|---|---|
| Maximal-Fluß | µg/cm2/h | 4,0 | 14,6 |
| t (Maximal-Fluß) | h | 15,1 | 4,5 |
| mittlerer Fluß (0-24h) | µg/cm2/h | 3,0 | 7,0 |
| mittlerer Fluß (24-48h) | µg/cm2/h | 1,6 | 4,3 |
| Dosis | µg/10µl | 477 | 469 |
| in 48 h resorbierte Dosis | µg | 75,4 | 183,0 |
| in 48 h resorbierte Dosis | % | 15,8 | 39,0 |

Der therapeutische Plasmaspiegel ($C_{ss}$) für die Behandlung von Parkinsonismus liegt bei 1-2 ng/ml, bei den anderen in der Tabelle genannten Indikationen ist der therapeutische Plasmaspiegel geringer.

Der perkutane Fluß (I), der bei einer gegebenen Fläche (A) benötigt wird, um den therapeutischen Plasmaspiegel zu erreichen, läßt sich aus der totalen Körper-Clearance (Cl) von LISURID wie folgt errechnen.

$$I = \frac{CL * C_{ss}}{A}$$

Wählt man für ein TDS eine Größe von 50 $cm^2$, so wird bei einer totalen Clearance für LISURID von 65250 ml/h mit einem perkutanen Fluß von 1.3-2.7 $\mu g/cm^2/h$ der gewünschte therapeutische Plasmaspiegel von 1-2 ng/ml erzielt. Die in vitro durch Maushaut erzielten Flüsse von bis zu über 14 $\mu g/cm^2/h$ liegen deutlich über dieser Anforderung. Auch bei realistischer Annahme einer um den Faktor 5 höheren Permeabilität der Maushaut für Lisurid wäre der benötigte transdermale Fluß beim Menschen gegeben.

Die erfindungsgemäßen Ergolin-Derivate enthaltenden Mittel zur transdermalen Applikation können zur Behandlung der gleichen Erkrankungen angewendet werden, wie die vorbekannten, beispielsweise oral oder subcutan zu applizierenden Mittel die Ergolin-Derivate enthalten.

Die nachfolgenden Ausführungsbeispiele dienen zur näheren Erläuterung der Erfindung. In ihnen wurden folgende Handelsprodukte verwendet:

Polyesterfolie von 0,074 mm Dicke (SkotchPak® 1009) des Herstellers 3M; Polypropylenfolie (Celgard® 2500) des Herstellers Celanese, Linerfolie SkotchPak® 1022 und 1360 vom Hersteller 3M; Transferkleber 9871 vom Hersteller 3M, Polyacrylester-Kleber vom Typ Gelva® 2723 des Herstellers Monsanto und Silikonklebstoff vom Typ X-7-4502 des Herstellers Dow Corning.

<u>Beispiel 1</u>

In 100 g einer 50 %igen Lösung von Silikonklebstoff in Benzin werden nacheinander

| | |
|---|---|
| 2.0 g | Lisurid und |
| 10,0 g | Isopropylpalmitat |

unter Rühren gelöst bzw. suspendiert (da die Klebstoffe trübe sind, läßt sich nicht klar entscheiden, ob eine vollständige Lösung vorliegt). Nach Entgasen des Ansatzes über 24 Stunden wird die Lösung/Suspension mittels einer Knife-over-Roll Beschichtungsvorrichtung auf fluorpolymerbeschichtete Polyesterfolie in der Weise aufgetragen, daß nach Entfernung des flüchtigen Lösemittels bei 60-80° C ein gleichmäßiger Film von 100 $g/m^2$ Feststoffauftrag entsteht. Anschließend wird mit einem lichtundurchlässigen Polyester-Abdeckfolie kaschiert. Das so erhaltene Laminat wird mittels einer Stanzvorrichtung in Einzelpflaster von 10 $cm^2$ Fläche geteilt und in einen aluminisierten Beutel luftdicht verpackt. Das Pflaster haftet nach Abziehen der Liner-Folie auf der Haut.

Die Gehaltsbestimmung ergibt eine gleichmäßige Wirkstoffverteilung von 4,4 $mg/cm^2$ im Mittel.

<u>Beispiel 2</u>

In 100 g einer 50 %igen Lösung von Polyacrylester-Klebstoff in Ethylacetat werden nacheinander

| | |
|---|---|
| 1,0 g | Lisurid und |
| 17,5 g | 1,2-Propandiol |

unter Rühren gelöst bzw. suspendiert. Nach Entgasen des Ansatzes über 24 Stunden wird die Lösung/Suspension mittels einer Knife-over-Roll Beschichtungsvorrichtung auf Polyesterfolie in der Weise aufgetragen, daß nach Entfernung der flüchtigen Lösemittel bei 60-80° C ein gleichmäßiger Film von 50 $g/m^2$ Feststoffauftrag entsteht. Anschließend wird mit einer lichtundurchlässigen Polyester Abdeck-Folie kaschiert. Das so erhaltene Laminat wird mittels einer Stanzvorrichtung in Einzelpflaster von 10 $cm^2$ Fläche geteilt und in einen aluminisierten Beutel luftdicht verpackt. Das Pflaster haftet nach Abziehen der Liner-Folie auf der Haut.

Der Gehalt an Ergolin-Derivat beträgt im Mittel 1,1 $mg/cm^2$.

<u>Beispiel 3</u>

Zu 100 g einer 50 %igen Lösung von Polyacrylester-Klebstoff in Ethylacetat werden nacheinander

| | |
|---|---|
| 1.0 g | Tergurid |
| 1,0 g | hochdisperse Kieselsäure und |

(fortgesetzt)

| | |
|---|---|
| 17,5 g | 1,2-Propandiol mit 10 % 1-Dodecanol |

unter Rühren gelöst bzw. suspendiert. Nach Entgasen des Ansatzes über 24 Stunden wird die Lösung/Suspension mittels einer Knife-over-Roll Beschichtungsvorrichtung auf einen silikonisierten Polyester-Liner in der Weise aufgetragen, daß nach Entfernung der flüchtigen Lösemittel bei 60-80° C ein gleichmäßiger Film von 100 g/m$^2$ Feststoffauftrag entsteht. Anschließend wird mit einer lichtundurchlässigen Polyester-Abdeck-Folie kaschiert. Das so erhaltene Laminat wird mittels einer Stanzvorrichtung in Einzelpflaster von 10 cm$^2$ Fläche geteilt und in aluminisierten Beutel luftdicht verpackt. Das Pflaster haftet nach Abziehen der Liner-Folie auf der Haut.

Der Gehalt an Tergurid liegt bei je 2,2 mg/cm$^2$.

Beispiel 4

Eine lichtundurchlässige Polyesterfolie von 7,4 cm Durchmesser wird mittels Zug und Wärme so verformt, daß eine runde Ausbuchtung von 10 cm$^2$ Fläche entsteht. Diese wird mit 1 ml einer Suspension von

| | |
|---|---|
| 3,0 mg | Protergurid |

in 1,2-Propandiol, welches 10 % Dexpanthenol enthält, gefüllt. Eine Polypropylen-Folie wird am Rand aufgeschweißt. Je nach Druck pro Zeiteinheit liegt die Siegeltemperatur zwischen 70° C und 100° C. Auf die durchlässige Polymerschicht wird Haftkleberfolie transferiert. Das Pflaster wird mit einem Liner versehen und in einem aluminisierten Beutel luftdicht verpackt.

## Patentansprüche

1. Transdermales therapeutisches System (TTS) zur Verabreichung von Lisurid oder dessen physiologisch unbedenklichem Salz, bestehend aus

   a) einer undurchlässigen Deckschicht,
   einer an der Deckschicht haftenden, das Ergolin-Derivat und penetrationsverstärkende Mittel enthaltenden, für diese Komponeneten durchlässigen Arzneimittelschicht,
   die selbsthaftend ist oder von einem Hauthaftkleber abgedeckt oder umgeben ist, welcher ebenfalls penetrationsverstärkende Mittel enthalten kann, und
   einer abziehbaren Schutzschicht oder
   b) einer undurchlässigen Deckschicht,
   einem an oder in der Deckschicht befindlichen, das Ergolin-Derivat und die penetrationsverstärkenden Mittel enthaltenden Arzneimittelreservoir,
   einer für diese Komponenten durchlässigen Polymerschicht,
   einer durchlässigen, gegebenfalls penetrationsverstärkende Mittel enthaltenden Hauthaftkleberschicht und
   einer abziehbaren Schutzschicht,

   worin das penetrationsverstärkende Mittel ein ein- oder mehrwertiger aliphatischer, cycloaliphatischer oder aromatisch-aliphatischer Alkohol mit bis zu 8 Kohlenstoffatomen, ein gesättigter oder ungesättigter Fettalkohol mit 8 bis 18 Kohlenstoffatomen, Mineralöl, eine gesättigte oder ungesättigte Fettsäure mit 8 bis 18 Kohlenstoffatomen, ein Isopropylester der Palmitinsäure, Isopalmitinsäure, Isostearinsäure oder Stearinsäure oder ein Dicarbonsäurediester der allgemeinen Formel

   $$R'OCO(CH_2)_mCOOR'$$

   ist, worin m eine Zahl von 4 bis 8 und R' jeweils einen Alkylrest mit maximal 6 Kohlenstoffatomen bedeuten, oder deren Gemisch ist.

2. TTS gemäss Anspruch 1, worin das penetrationsverstärkende Mittel aus der Gruppe der ein- oder mehrwertigen aliphatischen, cycloaliphatischen oder aromatisch aliphatischen Alkohole 1,2-Propandiol, Menthol, Dexpanthenol

und Benzylalkohol ist.

**3.** TTS gemäss Anspruch 1, worin das penetrationsverstärkende Mittel aus der Gruppe der gesättigten und ungesättigten Fettalkohole Laurylalkohol, Isocetylalkohol und Cetylalkohol ist.

**4.** TTS gemäss Anspruch 1, worin das penetrationsverstärkende Mittel aus der Gruppe der gesättigten und ungesättigten Fettsäuren Laurinsäure, Isopalmitinsäure, Isostearinsäure und Ölsäure ist.

**5.** TTS gemäss Anspruch 1, worin das penetrationsverstärkende Mittel aus der Gruppe der Dicarbonsäurediester Diisopropyladipat, Diisobutyladipat und Diisopropylsebacat ist.

**6.** Mittel zur transdermalen Applikation von Lisurid und dessen physiologisch unverträglichen Salzen auf Basis des transdermalen therapeutischen Systems gemäss den Patentansprüchen 1 - 5.

**7.** Mittel zur transdermalen Applikation von Lisurid und dessen physiologisch verträglichen Salzen auf Basis des transdermalen therapeutischen Systems gemäss den Patentansprüchen 1 - 5 zur Behandlung und Prävention des prämenstruellen Syndroms, zur Lactationshemmung und zur Behandlung des Parkinsonismus.

## Claims

**1.** A transdermal therapeutic system (TTS) for administering lisuride or its physiologically harmless salt consisting of

a) an impermeable covering layer, a pharmaceutical layer that adheres to said covering layer and contains the ergolin derivative and penetration-enhancing agents and is permeable to these components wherein said pharmaceutical layer adheres to the covering layer either by being self-adhesive or covered or encompassed by a skin adhesive that may also contain penetration-enhancing agents, and a detachable protective layer, or

b) an impermeable covering layer,
a drug reservoir that is located on or in said covering layer and contains the ergolin derivative and penetration-enhancing agents,
a polymer layer that is permeable to these components, a permeable polyacrylate skin adhesive layer that may optionally contain penetration-enhancing agents,
and a detachable protective layer

wherein the penetration-enhancing agent is a mono- or bivalent aliphatic, cycloaliphatic, or aromatic-aliphatic alcohol with up to 8 carbon atoms, a saturated or unsaturated fatty alcohol, mineral oil, a saturated or unsaturated fatty acid with 8 to 18 carbon atoms, an isopropyl ester of palmitic acid, isopalmitic acid, isostearic acid or stearic acid, or a dicarboxylic diester of the general formula

$$R'\ OCO\ (CH_2)_mCOOR'$$

where m is a number from 4 to 8 and R' each represent an alkyl residue with up to 6 carbon atoms or a mixture thereof.

**2.** The TTS according to claim 1 wherein the penetration-enhancing agent is selected from the group of mono- or multivalent aliphatic, cycloaliphatic, or aromatic aliphatic alcohols containing 1,2-propanediol, menthol, dexpanthenol, and benzyl alcohol.

**3.** The TTS according to claim 1 wherein the penetration-enhancing agent is selected from the group of saturated and unsaturated fatty alcohols containing lauryl alcohol, isocetyl alcohol, and cetyl alcohol.

**4.** The TTS according to claim 1 wherein the penetration-enhancing agent is selected from the group of saturated and unsaturated fatty acids containing lauric acid, isopalmitic acid, isostearic acid, and oleic acid.

**5.** The TTS according to claim 1 wherein the penetration-enhancing agent is selected from the group of dicarboxylic diesters containing diisopropyl adipate, diisobutyl adipate, and diisopropyl sebacate.

6. An agent for transdermal application of lisuride and its physiologically incompatible salts based on the transdermal therapeutic systems according to claims 1 to 5.

7. An agent for transdermal application of lisuride and its physiologically incompatible salts based on the transdermal therapeutic systems according to claims 1 to 5 for the treatment and prevention of premenstrual syndrome, for lactation inhibition and for the treatment of Parkinson's disease.

## Revendications

1. Système thérapeutique transdermique (STT) pour l'administration de lisuride ou de son sel physiologiquement inoffensif, consistant en

    a) une couche de recouvrement imperméable, une couche médicamenteuse adhésive à la couche de recouvrement contenant le dérivé d'ergoline et des agents intensifiant la pénétration, cette couche médicamenteuse étant auto-adhésive ou entourée ou recouverte d'un agent adhésif à la peau qui peut également contenir des agents intensifiant la pénétration, et une couche de protection pouvant être décollée ou
    b) une couche de recouvrement imperméable, un réservoir médicamenteux contenant le dérivé d'ergoline et les agents intensifiant la pénétration, se trouvant sur

    ou dans la couche de recouvrement, une couche polymère perméable pour ces composants, une couche d'agent adhésif à la peau, perméable, contenant éventuellement des agents intensifiant la pénétration, et une couche de protection pouvant être décollée,
    dans lequel l'agent intensifiant la pénétration est un alcool aliphatique mono- ou polyvalent, cycloaliphatique ou aromatique-aliphatique avec jusqu'à 8 atomes de carbone, un alcool gras saturé ou insaturé avec 8 à 18 atomes de carbone, de l'huile minérale, un acide gras saturé ou insaturé avec 8 à 18 atomes de carbone, un ester isopropylique de l'acide palmitique, de l'acide isopalmitique, de l'acide isostéarique ou de l'acide stéarique, ou un diester d'acide dicarboxylique de formule générale $R'OCO(CH_2)_mCOOR'$, dans laquelle m est un chiffre allant de 4 à 8 et R' est chaque fois un radical alkyle avec 6 atomes de carbone maximum, ou un mélange de ceux-ci.

2. STT selon la revendication 1, dans lequel l'agent intensifiant la pénétration issu du groupe des alcools aliphatiques mono ou polyvalents, cycloaliphatiques ou aromatiques-aliphatiques est le 1,2-propanediol, le menthol, le dexpanthénol et l'alcool benzylique.

3. STT selon la revendication 1, dans lequel l'agent intensifiant la pénétration issu du groupe des alcools gras saturés ou insaturés est l'alcool laurique, l'alcool isocétylique et l'alcool cétylique.

4. STT selon la revendication 1, dans lequel l'agent intensifiant la pénétration issu du groupe des acides gras saturés ou insaturés est l'acide laurique, l'acide isopalmitique, l'acide isostéarique et l'acide oléique.

5. STT selon la revendication 1, dans lequel l'agent intensifiant la pénétration issu du groupe des diesters d'acide dicarboxylique est l'adipate de diisopropyle, l'adipate de düsobutyle et le sébaçate de diisopropyle.

6. Agent pour l'application transdermique de lisuride et de ses sels incompatibles physiologiquement, à base du système thérapeutique transdermique selon les revendications 1 à 5.

7. Agent pour l'application transdermique de lisuride et de ses sels compatibles physiologiquement, à base du système thérapeutique transdermique selon les revendications 1 à 5, pour le traitement et la prévention du syndrome prémenstruel, pour l'inhibition de la lactation et pour le traitement de la maladie de Parkinson.